# EUROPEAN PATENT APPLICATION

(11) **EP 4 830 691 A1**
(43) Date of publication of application: **28.10.2026**
(21) Application number: 26173275.4
(22) Date of filing: 20.04.2026
(51) Int. Cl.: A61K 6/30, A61C 7/14, A61K 6/62, A61K 6/76, A61K 6/887

(54) **ORTHODONTIC COMPOSITIONS, SYSTEMS, AND METHODS OF USING SAME**

(30) Priority: 25.04.2025 US 202563794508 P
(71) Applicant: Ormco Corporation, Brea, CA 92821 (US)
(72) Inventor: Mendieta, Marla, Brea, 92821 (US)
(74) Representative: Cleveland Scott York

(57) **Abstract**

A photo-curable orthodontic adhesive composition is provided. The photo-curable orthodontic adhesive composition includes a first catechol methacrylate monomer; at least one ethylenically unsaturated monomer comprising two or more methacrylate groups; a photoinitiator system; and a first filler. Further provided is an orthodontic adhesive system for adhering an orthodontic device to a tooth is provided. The orthodontic adhesive system comprising: a) a bonding primer composition comprising a second catechol methacrylate monomer and a volatile solvent; b) a bonding sealant composition comprising at least 25 weight percent of 2-hydroxyethyl methacrylate and a second filler; and c) the photo-curable orthodontic adhesive composition.

## Description

### CROSS REFERENCE TO RELATED APPLICATION

This application claims the benefit of U.S. Provisional App. No. 63/794,508, filed on April 25, 2025, which is hereby incorporated herein by reference in its entirety.

### TECHNICAL FIELD

The present invention relates generally to orthodontic compositions and, more particularly, to orthodontic adhesives, adhesive systems, and methods of using those adhesives and systems.

### BACKGROUND

Current orthodontic treatment with orthodontic brackets or other devices that are attached to the patient's teeth may require the enamel to be prepared prior to attachment of the device to the tooth. Preparation of the tooth surface may be through a series of steps including cleaning, acid etching, and sealing, with intermediate rinse and dry steps, before the clinician may apply an adhesive. For example, to bond a bracket to tooth enamel, each tooth is first cleaned with a slurry of abrasive, such as pumice, to remove pellicle from the enamel. Then, after rinsing and drying the cleaned surface, a phosphoric acid etchant is carefully placed on the surface locations of the tooth to which the clinician desires to attach the orthodontic device. The acid etching step demineralizes the enamel surface and removes a layer of approximately 30 µm or so of hydroxyapatite from the enamel rods. After between 15 and 90 seconds of etch time, the etchant is rinsed away with a water spray and a high flow evacuator. In this way, etching provides a porous structure.

Following the drying step after etching, a sealant is applied to the etched surface. The sealant may penetrate the porous, acid etched surface. Once the sealant cures, a mechanical interlock is created between the tooth and the sealant via the porous structure. An adhesive and the bracket may be pressed onto the sealed surface with the adhesive between the bracket and the sealant. The adhesive may be a composite resin paste adhesive that includes a mixture of methacrylate monomers, a photo-initiator, and a glass/hydroxyapatite powder. Once the adhesive cures, it secures the bracket to the sealant. This bonding arrangement results in a sandwich-like construction with the sealant and the adhesive sandwiched between the tooth surface and the orthodontic bracket. This procedure and bonding arrangement is then repeated for each tooth that will receive an orthodontic device and so, in the case of orthodontic brackets and molar tube, this may involve 28 teeth per patient.

The current preparation process has many drawbacks. From the perspective of the clinician, it is a manually time-intensive process. It is not surprising that office chair time during the entire bonding procedure is lengthy. Overall, bonding orthodontic brackets to teeth is costly. From the patient's perspective, the process is uncomfortable and enamel removal is often irreversible due to the difficulty of remineralizing dental hard tissues. Thus, the tooth surface may be permanently compromised by acid etching.

Furthermore, certain patients may have an allergic reaction to the etchant. Liquid etchant may flow to the gingiva where it may irritate the soft tissue. Gel etchant, despite allowing more precise placement, requires skillful application and is more difficult to remove. In either application, when the etchant must be rinsed away, care must be taken not to splash or wash the etchant in a manner that may harm the patient or clinician, but the rinsing must be thorough so that the etching reaction is terminated and there is no residual acid or mineral debris that hinders the mechanical interlock between the tooth and the device.

During treatment, the decalcification of the enamel surface adjacent to fixed orthodontic appliances is prevalent. Decalcification is manifested as a white spot lesion (WSL). If left untreated, WSL may progress to produce carious cavitations and may also present aesthetic problems. Thus, the prevention, diagnosis, and treatment of WSLs is crucial to minimize tooth decay as well as tooth discoloration that could compromise the aesthetics of the patient's smile. However, the problems and costs don't end with bonding.

Therefore, a need exists for orthodontic compositions, including adhesives, adhesive systems, and methods of using those compositions and systems, that do not require the complex pre-attachment treatment described above and that reduce issues associated with debonding orthodontic devices from teeth.

### SUMMARY

Certain aspects of the present disclosure are described in the appended claims. There are additional features and advantages of the subject matter described herein. They will become apparent as this specification proceeds. In this regard, it is to be understood that the claims serve as a brief summary of varying aspects of the subject matter described herein. The various features described in the claims and below for various embodiments may be used in combination or separately. For example, specified ranges may be inclusive of their recited endpoints, unless explicitly excluded. Any particular embodiment need not provide all features noted above, nor solve all problems or address all issues noted above.

According to embodiments of the present invention, a photo-curable orthodontic adhesive composition is provided. The photo-curable orthodontic adhesive composition comprising a first catechol methacrylate monomer; at least one ethylenically unsaturated monomer comprising two or more methacrylate groups; a photo-initiator system; and a first filler.

According to an embodiment, an orthodontic adhesive system for adhering an orthodontic device to a tooth is provided. The orthodontic adhesive system comprising:
a) a bonding primer composition comprising a second catechol methacrylate monomer and a volatile solvent;
b) a bonding sealant composition comprising at least 25 weight percent of 2-hydroxyethyl methacrylate and a second filler; and
c) the photo-curable orthodontic adhesive composition,
wherein the orthodontic adhesive system is void of any etchant composition.

According to another embodiment, a method of adhering an orthodontic device to a tooth is provided. The method comprising:
applying a bonding primer composition to a cleaned surface of the tooth, wherein the bonding primer composition comprising a second catechol methacrylate monomer and a volatile solvent to form a primer layer on the cleaned surface of the tooth;
applying a bonding sealant composition to the primer layer to form a sealant layer thereon, wherein the bonding sealant composition comprising at least 25 weight percent of 2-hydroxyethyl methacrylate, and a second filler;
applying the photo-curable orthodontic adhesive composition to the sealant layer previously applied to the primer layer, and/or applying the photo-curable orthodontic adhesive composition to the orthodontic device; and
affixing the orthodontic device to the tooth with the primer layer, sealant layer, and the photo-curable orthodontic adhesive composition situated between the tooth and the orthodontic device.

According to a first aspect of the present invention there is provided a photo-curable orthodontic adhesive composition comprising:
a catechol methacrylate monomer;
at least one ethylenically unsaturated monomer comprising two or more methacrylate groups;
a photo-initiator system; and
a first filler.

Preferably the catechol methacrylate monomer comprises 10-((3-(3,4-dihydroxyphenyl)propanoyl)oxy)decyl methacrylate.

Preferably the at least one ethylenically unsaturated monomer comprising two or more methacrylate groups is selected from the group consisting of 2,2-bis-(4-(2-hydroxy-3-methacryloyloxypropoxy) phenyl) propane (Bis-GMA), triethyleneglycol dimethacrylate (TEGDMA), ethylene glycol dimethacrylate (EGDMA), ethoxylate bisphenol A dimethacrylate (Bis-EMA), urethane dimethacrylate (UDMA), anhydrous 4-methacryloxyethyl trimellitic anhydride (4-META), dipentaerythritol pentaacrylate monophospate, biphenyl dimethacrylate (BPDM), glycerolphosphate dimethacrylate (GPDM), propylene glycol dimethacrylate (PGDMA), and combinations thereof.

Preferably the at least one ethylenically unsaturated monomer comprising two or more methacrylate groups comprises a combination of 2,2-bis-(4-(2-hydroxy-3-methacryloyloxypropoxy) phenyl) propane (Bis-GMA) and ethoxylate bisphenol A dimethacrylate (Bis-EMA).

Preferably the photo-initiator system comprising a ketone sensitizer, an amine donor, and optionally an iodonium salt.

Preferably the ketone sensitizer is selected from the group consisting of camphorquinone, 1-phenyl propane-1,2-dione, benzil, diacetyl, benzyl dimethyl ketal, benzyl diethyl ketal, benzyl di(2-methoxyethyl)ketal, 4,4,'-dimethylbenzyl dimethyl ketal, anthraquinone, 1-chloroanthraquinone, 2-chloroanthraquinone, 1,2-benzanthraquinone, 1-hydroxyanthraquinone, 1-methylanthraquinone, 2-ethylanthraquinone, 1-bromoanthraquinone, thioxanthone, 2-isopropyl thioxanthone, 2-nitrothioxanthone, 2-methyl thioxanthone, 2,4-dimethyl thioxanthone, 2,4-diethyl thioxanthone, 2,4-diisopropyl thioxanthone, 2-chloro-7-trifluoromethyl thioxanthone, thioxanthone-10,10-dioxide, thioxanthone-10-oxide, diphenyliodonium hexafluorophosphate, p-octyloxy-phenylphenyl iodonium hexafluoroantimonate, benzoin methyl ether, benzoin ethyl ether, isopropyl ether, benzoin isobutyl ether, benzophenone, bis(4-dimethylaminophenyl)ketone, 4,4,'-bisdiethylaminobenzophenone, and combinations thereof.

Preferably the amine donor comprises a secondary or tertiary amine selected from the group consisting of triethanolamine, diethanolamine, methyl diethanolamine, 2-dimethylaminoethyl (meth)acrylate, 3-dimethylaminopropyl (meth)acrylate, 4-dimethylaminobutyl (meth)acrylate, 6-dimethylaminohexyl (meth)acrylate, 10-dimethylaminodecyl (meth)acrylate, 4-dimethylaminophenetyl alcohol, 2-(4-(dimethylamino)phenyl)ethanol, 2-(4-(dipropylamino)phenyl)ethanol, 2-ethylhexyl-4-(dimethylamino) benzoate, N,N-di(2-hydroxyethyl)-p-toluidine, N,N-di(2-hydroxypropyl)-p-toluidine, N,N-dimethyl-p-toluidine, N,N-dipropyl-p-toluidine, N,N-diethyl-p-toluidine, N,N-diethoxyethyl-p-toluidine, N,N-dibutoxy ethyl-p-toluidine, N,N-di(polyoxyethylene)oxyethyl-p-toluidine, hexamethylenediamine, a dimethylamine aqueous solution, pentamethylenediamine, diethylamine, ethylenediamine, 2-aminoethanol, triethylamine, 2-dimethylaminoethanol, and combinations thereof.

Preferably the iodonium salt is present and selected from the group consisting of tolylcumyliodonium tetrakis(pentafluorophenyl)borate, tolylcumyliodonium tetrakis(3,5-bis(trifluoromethyl)-phenyl)borate, diphenyliodonium chloride, diphenyliodonium hexafluorophosphate, diphenyliodonium hexafluoroantimonate, diphenyliodonium tetrafluoroboarate, and combinations thereof.

Preferably the first filler is selected from the group consisting of inorganic metal, salt, oxide, fluoride, or nitride; silicate glass; aluminosilicate glass; aluminoborosilicate glass; fluoroaluminosilicate glass; barium-containing glass; quartz; colloidal silica; precipitated silica; zirconia-silica; mixed oxides fused together; polymeric filler; polymerized composite fillers with inorganic particles; and combinations thereof.

Preferably first filler comprises a barium-containing glass filler comprising:
a first barium glass filler having an average particle diameter in a range of about 0.5 to about 0.9 µm;
a second barium glass filler having an average particle diameter in a range of about 1.7 to about 2.3 µm; and
a third barium glass filler having an average particle diameter in a range from about 8 to about 10 µm.

Preferably the catechol methacrylate monomer comprises 10-((3-(3,4-dihydroxyphenyl)propanoyl)oxy)decyl methacrylate;
wherein the at least one ethylenically unsaturated monomer comprising two or more methacrylate groups comprising a combination of 2,2-bis-(4-(2-hydroxy-3-methacryloyloxypropoxy) phenyl) propane (Bis-GMA) and ethoxylate bisphenol A dimethacrylate (Bis-EMA);
wherein the photo-initiator system comprising a ketone sensitizer, an amine donor, and an iodonium salt; and
wherein the first filler comprising a barium-containing glass filler comprising:
   a first barium glass filler having an average particle diameter in a range of about 0.5 to about 0.9 µm;
   a second barium glass filler having an average particle diameter in a range of about 1.7 to about 2.3 µm; and
   a third barium glass filler having an average particle diameter in a range from about 8 to about 10 µm.

Preferably the photo-initiator system comprising camphorquinone, 2-ethylhexyl-4-(dimethylamino) benzoate, and diphenyliodonium hexafluorophosphate.

Preferably the photo-curable orthodontic adhesive composition comprises:
0.01 to 1.0 weight percent of the catechol methacrylate monomer;
5.0 to 25.0 weight percent of the at least one second ethylenically unsaturated monomer comprising two or more methacrylate groups;
0.1 to 5.0 weight percent of the second photo-initiator system; and
50.0 to 80.0 weight percent of the first filler,
wherein each weight percent is based on the entire weight of the photo-curable orthodontic adhesive composition.

Preferably the orthodontic adhesive system for adhering an orthodontic device to a tooth comprises:
a) a bonding primer composition comprising a second catechol methacrylate monomer and a volatile solvent;
b) a bonding sealant composition comprising at least 25 weight percent of 2-hydroxyethyl methacrylate and a second filler; and
c) the photo-curable orthodontic adhesive composition according to any one of claims 1-13,
wherein the orthodontic adhesive system is void of any etchant composition.

Preferably the first and second catechol methacrylate monomer are the same or different.

Preferably the first and second catechol methacrylate monomer comprises 10-((3-(3,4-dihydroxyphenyl)propanoyl)oxy)decyl methacrylate.

According to a second aspect of the present invention there is provided an orthodontic adhesive system for adhering an orthodontic device to a tooth, comprising:
a syringe comprising the photo-curable orthodontic adhesive composition according to the first aspect of the present invention;
a first container comprising a bonding primer composition comprising a second catechol methacrylate monomer and a volatile solvent;
a second container comprising a bonding sealant composition comprising at least 25 weight percent of 2-hydroxyethyl methacrylate, and a second filler; and
optionally, a brush applicator and/or an orthodontic device.

According to a third aspect of the present invention there is provided a method of adhering an orthodontic device to a tooth with an orthodontic adhesive system including a bonding primer composition, a bonding sealant composition, and the photo-curable orthodontic adhesive composition according to the first aspect of the present invention, the method comprising:
applying the bonding primer composition to a cleaned surface of the tooth, wherein the bonding primer composition comprising a second catechol methacrylate monomer and a volatile solvent to form a primer layer on the cleaned surface of the tooth;
applying the bonding sealant composition to the primer layer to form a sealant layer thereon, wherein the bonding sealant composition comprising at least 25 weight percent of 2-hydroxyethyl methacrylate, and a second filler;
applying the photo-curable orthodontic adhesive composition to the sealant layer previously applied to the primer layer, and/or applying the photo-curable orthodontic adhesive composition to the orthodontic device; and
affixing the orthodontic device to the tooth with the primer layer, sealant layer, and the photo-curable orthodontic adhesive composition situated between the tooth and the orthodontic device.

Preferably the method further comprises:
performing prophylaxis on the surface of the tooth with an abrasive pumice prior to applying the first layer; and
removing the abrasive pumice from the surface of the tooth by rinsing with water and drying the tooth.

Preferably the first and second catechol methacrylate monomer are the same or different.

### BRIEF DESCRIPTION OF THE DRAWINGS

The accompanying drawing, which are incorporated in and constitute a part of this specification, illustrate embodiments of the invention and, together with a general description of the invention given above, and the detailed description given below, serve to explain the invention. It will be appreciated that for purposes of clarity and where deemed appropriate, reference numerals have been repeated in the figures to indicate corresponding features.
FIG. 1 shows a set of orthodontic brackets with individual brackets attached to the teeth of a patient;
FIG. 2 is a cross-sectional view taken along section line 2 - 2 of FIG. 1;
FIG. 3 is an enlarged view of an encircled area 3 of FIG. 2 according to one embodiment of the invention;
FIG. 4 is a perspective view of one embodiment of the invention with attachments of one embodiment of the invention attached to the teeth of a patient to facilitate orthodontic treatment with an aligner; and
FIG. 5 shows a prior art method for adhering an orthodontic device to a tooth,
FIG. 6 show a method of adhering an orthodontic device to a tooth, in accordance with an embodiment of the invention.
FIG. 7 shows an orthodontic adhesive system, in accordance with an embodiment of the invention.

### DETAILED DESCRIPTION

To address these and other issues, a clinician may utilize an orthodontic adhesive system **10** to adhere an orthodontic device to a patient's tooth. As described in detail below, the orthodontic adhesive system **10** includes a photo-curable orthodontic adhesive composition comprising a catechol methacrylate monomer. The orthodontic adhesive system **10** may further include a bonding primer composition and a bonding sealant composition. The compositions may be separately applied to a tooth and so form individual layers, one layer on the other, described below. The combination of the photo-curable orthodontic adhesive composition, the bonding primer composition, and the bonding sealant composition may be provided as a kit, along with instructions for use thereof.

According to the embodiments of the invention, the orthodontic adhesive system **10** may eliminate one or more of the tooth preparation steps described above. For example, the orthodontic adhesive system **10** may not require one or more of the cleaning and/or acid etching steps, described above, though the system **10** secures the orthodontic bracket **12** to a corresponding tooth **14.** As such, the orthodontic adhesive system **10** will eliminate or minimalize demineralization issues created by acid etching during preparation of the tooth surface. The orthodontic adhesive system **10** according to embodiments of the invention may have self-healing properties so that the orthodontic adhesive system **10** resists aging and long-term degradation.

A complicating factor for orthodontic adhesives is the environment to which the adhesive is exposed. The mouth of the patient is filled with saliva, which is an aqueous solution of electrolytes, enzymes, and cellular matter. This environment necessitates the complicated tooth preparation process and bonding process, described above, to produce a mechanical bond between the tooth and the orthodontic device.

The oral environment has similarities to seawater, which is a solution of water, electrolytes, and biological material. In the ocean, mussels possess a remarkable ability to attach and detach themselves from surfaces that are submersed in seawater. Marine mussels secrete a glue-like sticky material, known as byssus, which is responsible for the strong adhesion to rocks and other surfaces in turbulent marine environment. The byssus is a proteinaceous bundle of thread-like materials that spreads out in a radially outward direction. It consists of four parts, namely, plaque, thread, stem, and root. In other words, mussel byssus is a protein derived from marine mussels. Byssal threads are attached to the root at the base of mussel foot where a combination of 12 retractor muscles controls the tension in them. More than 25 different mussel foot proteins (mfp) have been identified in byssus, out of which 5 (mfp-2 to mfp-6) are unique to plaque. These 5 mfp have a high content of the usually rare modified amino acid 3,4-dihydroxy-L-phenylalanine (hereinafter "DOPA").

DOPA includes a catechol moiety. When combined with oxidant cations from seawater under basic pH conditions, catechol oxidation of the catechol moiety of DOPA produces quinine. The quinine can form a cross-linked polymer matrix in the bonding network. Further, when bonding to rocks, the catechol moiety of DOPA may undergo chelation with inorganic oxides found in the rock. Cohesion between molecules of DOPA is aided by multivalent cations, such as Fe³⁺ and Ca²⁺ ions. These cations form metal complexes between non-oxidized catechols of DOPA and facilitate wet adhesion of the bonding network in seawater. It has been found that it is the catechol functionality of DOPA that gets attached with external surface during the adhesion process and so at least facilitates the adhesion of the mussel to a variety of substrates, including wood, metal, and mineral surfaces, among others, when submerged in seawater.

Applicants have found that using a marine mussel protein mimic as a component in the orthodontic adhesive system **10** will provide sufficient bond strength between an orthodontic device, such as the orthodontic bracket **12,** and a tooth **14.** Bonding may be accomplished in the absence of the complicated preparation and bonding process described above.

In accordance with an embodiment of the invention, organic compounds suitable to function as a marine mussel protein mimic include a monomer having a catechol moiety and/or a catechol derivative moiety and therefore have similar properties to DOPA present in mussel foot proteins. The catechol moiety and/or the catechol derivative moiety is covalently bonded via a linking group to an ethylenically unsaturated moiety, such as a methacrylate group. Thus, in accordance with an embodiment, the orthodontic adhesive system **10** includes a catechol methacrylate (CMA) monomer having a general Formula (I):

Ar-L-E (I),

where Ar is a catechol moiety or a catechol derivative moiety; L is a linking group; and E is an ethylenically unsaturated moiety. In an embodiment, Ar may comprise 3,4-dihydroxyphenylpropionic acid, (3-(3,4-dihydroxyphenyl)-2-methylpropanonoic acid, or DOPA. In an embodiment, L may comprise a diol selected from ethylene glycol, propylene glycol, butylene glycol, polyethylene glycols, hexylene glycols, octylene glycols, decylene glycols. In an embodiment, E may comprise an acrylate or methacrylate group. Non-limiting examples of the catechol methacrylate (CMA) monomer include 4-[3-(3,4-dihydroxyphenyl)propionoxy]butyl methacrylate (C4-CMA); 6-[3-(3,4-dihydroxyphenyl)propionoxy]hexyl methacrylate (C6-CMA); 8-[3-(3,4-dihydroxyphenyl)propionoxy]octyl methacrylate (C8-CMA); and/or 10-((3-(3,4-dihydroxyphenyl)propanoyl)oxy)decyl methacrylate (C10-CMA).

In one embodiment, the CMA monomers may be synthesized by reacting the catechol moiety (e.g., 3,4-dihydroxyphenylpropionic acid or 3-(3,4-dihydroxyphenyl)-2-methylpropanonoic acid) with a hydroxyalkyl methacrylate (e.g., hydroxyethyl methacrylate or hydroxydecyl methacrylate) in the presence of a coupling agent (e.g., 1-ethyl-3-(3-dimethylaminopropyl)carbodiimide (EDC) and N-hydroxysuccinimide (NHS)).

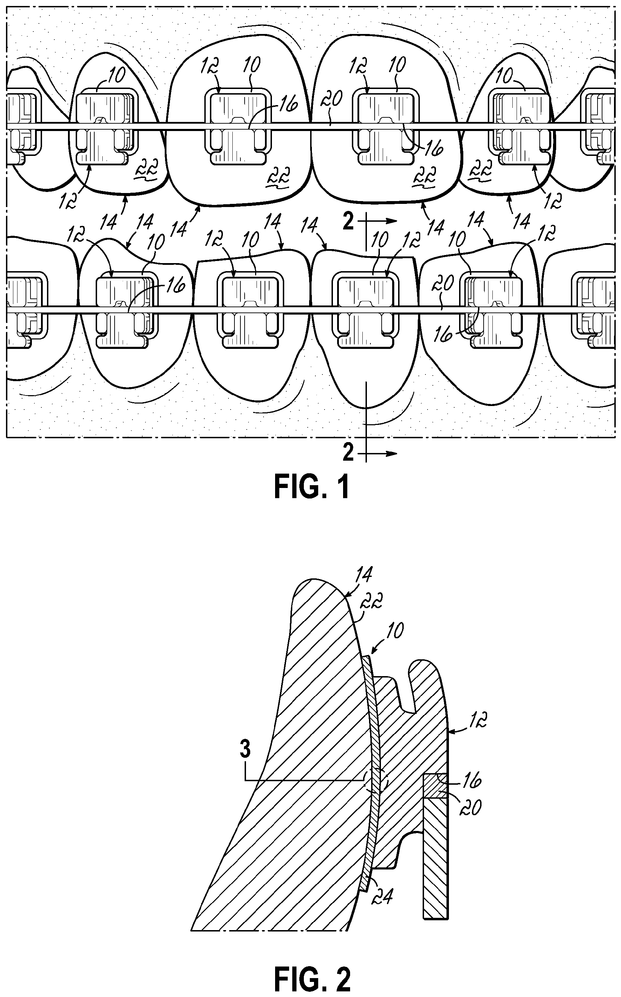

By way of example only, as shown in FIG. 1, an orthodontic bracket **12** may be used in an orthodontic procedure. One orthodontic bracket **12** may be affixed to each of a plurality of teeth **14** with the orthodontic adhesive system **10.** The orthodontic bracket **12** defines a substantially transversely disposed archwire slot **16,** which receives an archwire **20.** The orthodontic bracket **12** may be adhesively secured to an exterior facing surface **22** of the tooth **14** with the orthodontic adhesive system **10.** Although not shown in FIG. 1, the orthodontic adhesive system **10** may be between each of the orthodontic brackets **12** and the corresponding tooth **14.** While brackets **12** are shown and described herein, embodiments of the present invention may be utilized to bond other orthodontic appliances to the patient's teeth. For example, the orthodontic adhesive system **10** may be utilized to bond a lingual retainer, bite turbos, or aligner attachments (specifically, made of the photo-curable orthodontic adhesive composition) to name a few, to the patient's teeth **14.**

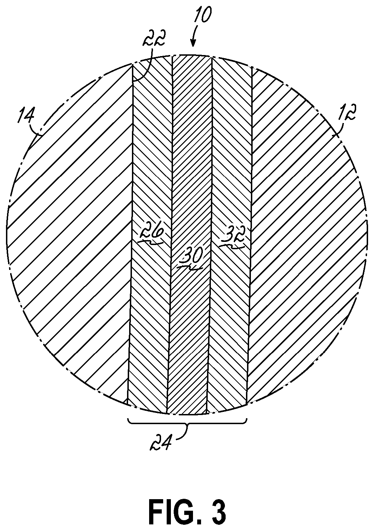

With reference to FIG. 2, the orthodontic adhesive system **10** may include a plurality of layers **24** of individual, separately-applied components, as is shown in FIG. 3. While the plurality of layers **24** appear to be illustrated in equal parts (layers 26, 30, and 32 in FIG. 3), this is not necessary to the invention. In accordance with the present invention, the plurality of layers **24** of individual, separately-applied components may be of differing dimensions and thicknesses in relation to each other. The plurality of layers **24** include one or more components that are configured to bond to one of the tooth surface **22** or an orthodontic appliance **12** or form a bond between other components in a sandwich-like composite construction. When attached to respective teeth **14** with the orthodontic adhesive system **10,** the brackets **12** and the archwire **20** collectively provide orthodontic treatment.

With further reference to FIG. 3, the plurality of layers **24** includes three layers, layer **26,** layer **30,** and layer **32.** In an embodiment, the layer **26** comprises a "surface primer" or "bonding primer" composition in direct contact with the tooth surface **22;** the layer **30** comprises a "sealant" or "bonding sealant" composition applied to the layer **26;** and layer **32** comprises a photo-curable orthodontic adhesive composition. Any single one, a combination of, or each layer **26, 30,** and **32** is a mixture that includes the catechol methacrylate (CMA) monomer having the general Formula (I). When more than one layer comprises the catechol methacrylate (CMA) monomer, it may be the same or different CMA monomer.

In the case of the layer **32,** which may include the photo-initiator system, the orthodontic bracket **12** may be pressed against the composite layer **26, 30,** and **32** shown in FIG. 3. The adhesive layer **32** may then be cured by exposing it to light, such as visible blue light (e.g., wavelengths of about 450 nm to about 475 nm). This photo-curing process cures at least the layer **32.** By way of further example, each of the layers **26, 30,** and **32** may be cured at the same time or at different times. The timing of each cure depends on the preferences of the clinician. In the case of the layer **30,** which may include a photo-initiator system. a clinician may prefer to partially cure the layer **30** to make it tackier and then apply the remaining layer with a final cure of each of the layers **26, 30,** and **32** together. When the layers **26, 30,** and **32** are cured, the orthodontic adhesive system **10** bonds the orthodontic bracket **12** to the tooth surface **22.**

Referring to the layer **26** of FIG. 3, unlike some conventional orthodontic primers, the catechol or catechol derivative moiety forms adhesion networks through hydrogen bonding and metal-ligand complexes with hydroxyapatite without one or more of cleaning, etching, or drying preparation steps. Additionally, the catechol or catechol derivative moieties may undergo Michael addition with collagen in enamel or in dentin to chemically bond the layer **26** to the tooth surface **22.**

In accordance with an embodiment, the bonding primer composition used for forming the layer **26** comprises a mixture including a compound having the general Formula (I). As an example, the bonding primer composition may comprise one or more catechol methacrylate (CMA) monomers having a general Formula (I) and a volatile solvent. The one or more catechol methacrylate monomers may be present in the bonding primer composition in an amount ranging from about 0.001% (w/w) to about 1% (w/w), such as from about 0.002% (w/w) to about 0.5% (w/w) or from about 0.005% (w/w) to about 0.1% (w/w).

The bonding primer composition may further include one or more acidic polymerizable monomers having at least one ethylenically unsaturated group and at least one acidic moiety. The acidic moiety can be any acidic functional group. Examples of acidic moieties include, but are not limited to, sulfonic acid, sulfinic acid, carboxylic acid, carboxylic acid anhydride, phosphonic acid or its derivative, and phosphoric acid or its derivative, with a derivative being a salt or ester of the respective acid.

In one embodiment, the acidic polymerizable monomer contains at least one acidic moiety selected from the group consisting of phosphonic acid or its derivative and phosphoric acid or its derivative. Examples includes, but are not limited to, phenyl methacryloxyethyl phosphate (MEP), glyceryldimethacrylate phosphate (GPDM), dipentaerithritol pentaacrylate phosphate (PENTA), methacryloyloxybutyl phosphate (MBP), methacryloyloxyhexyl phosphate (MHP), methacryloyloxydecyl phosphate (MDP), hydroxyethylmethacrylate phosphate (HEMAP), and bis(hydroxyethylmethacrylate) phosphate (BMEP), and any combination thereof.

In another embodiment, the acidic monomer contains at least one acidic moiety selected from the group consisting of carboxylic acid and carboxylic anhydride. Examples includes, but are not limited to, maleic acid, itaconic acid, methacrylic acid, acrylic acid, polymerizable homopolymer or copolymer of an α,β-unsaturated carboxylic acid such as (meth)acrylated poly(acrylic acid), (meth)acrylated poly(acrylic acid) copolymer such as (meth)acrylated poly(acrylic acid-maleic acid) copolymer or (meth)acrylated poly(acrylic acid-maleic acid-itaconic acid) copolymer, maleic anhydride, 4-methacryloxyethyltrimellitic anhydride, 4-methacryloxyethyltrimellitic acid, any addition product of mono- or dianhydride compound with an hydroxyalkylmethacrylate compound such as the addition product of pyromellitic acid anhydride and 2-hydroxyethyl methacrylate, the addition product of pyromellitic acid anhydride and glycerol dimethacrylate, the addition product of 3,3',4,4'-benzophenonetetracarboxylic dianhydride and hydroxyethyl methacrylate, the addition product of phthalic anhydride and hydroxyethyl methacrylate, the addition product of maleic anhydride and glycerol dimethacrylate, and any combination thereof.

The concentration of the one or more acidic polymerizable monomers ranges from about 1% (w/w) to about 30% (w/w) of the composition, such as from about 3% (w/w) to about 20% (w/w) or from about 5% (w/w) to about 10% (w/w) of the composition.

In one embodiment, the bonding primer composition further includes a volatile solvent. Nonlimiting examples of useful solvents include methanol, ethanol, isopropanol, acetone, ethyl acetate, methyl ethyl ketone, or combinations thereof. Water may be present in the bonding primer composition as a co-solvent.

The bonding primer composition may further include one or more stabilizers. Examples of stabilizers include, but are not limited to, butylated hydroxytoluene (BHT), hydroquinone, hydroquinone monomethyl ether (MEHQ), 3,5-di-tert-butyl-4-hydroxyanisole (2,6-di-tert-butyl-4-ethoxyphenol), 2,6-di-tert-butyl-4-(dimethylamino)methylphenol or 2,5-di-tert-butyl hydroquinone, 2-(2'-hydroxy-5'-methylphenyl)-2H-benzotriazole, 2-(2'-hydroxy-5'-t-octylphenyl)-2H-benzotriazole, 2-hydroxy-4-methoxybenzophenone (UV-9), 2-(2'-hydroxy-4',6'-di-tert-pentylphenyl)-2H-benzotriazole, 2-hydroxy-4-n-octoxybenzophenone, and 2-(2'-hydroxy-5'-methacryloxyethylphenyl)-2H-benzotriazole. The stabilizer(s) may be present in an amount of from about 0 to about 5% (w/w), such as from about 0.001% (w/w) to about 2% (w/w), or from 0.01% (w/w) to about 1% (w/w) . In accordance with an embodiment, a typical formulation for the bonding primer composition is shown in Table 1, below.

**Table 1: Typical Bonding Primer Composition.**

| **Chemical** | **Example** | **Weight %** |
|---|---|---|
| CMA | C10-CMA | 0.01 to 1 |
| Acidic monomer | MDP | 1 to 30 |
| Stabilizer | BHT | 0 to 5 |
| Volatile solvent | Acetone | 60 to 95 |
| Water | Deionized water | 0 to 10 |

In an embodiment, the bonding primer composition comprises 7.5% (w/w) of acidic monomer, 0.005% (w/w) of CMA, 0.0075% (w/w) of stabilizer, in a balance of acetone.

The bonding primer composition may be applied to the tooth surface **22** with a brush to form layer **26.** While not detrimental to the orthodontic adhesive system **10,** the tooth surface **22** does not require other preparation techniques such as etching. But it may be advantageous to perform prophylaxis on the tooth surface **22** with an abrasive pumice prior to applying the bonding primer composition as layer **26;** and removing the abrasive pumice from the surface of the tooth by rinsing with water and drying the tooth.

In an embodiment and with reference to FIG. 3, the layers **30** and **32** may be separately applied on the layer **26,** previously applied to the tooth surface **22.** The bonding sealant composition may be applied directly to layer **26** to form layer **30.** The layer **30** may be in direct contact and may chemically bond with the catechol and/or catechol derivative-containing compound within the layer **26,** before or after that layer cures.

In the embodiment shown in FIG. 3, the layer **30** may include a (meth)acrylate-based resin comprising an ethylenically unsaturated functional group. As used herein, (meth)acrylate may mean acrylate or methacrylate, which is a moiety comprising an ethylenically unsaturated functional group that can react and bond to the layer **26.** Examples of ethylenically unsaturated monomers include, but are not limited to hydroxyethyl(meth)acrylate hydroxypropyl (meth)acrylate, hydroxybutyl (meth)acrylate, glycerol di(meth)acrylate, glycerol mono(meth)acrylate, methyl(meth)acrylate, ethyl(meth)acrylate, propyl(meth)acrylate, butyl(meth)acrylate, hexyl(meth)acrylate, octyl(meth)acrylate, lauryl(meth)acrylate, decyl(meth)acrylate, tridecyl(meth)acrylate, 2-ethoxyethyl(meth)acrylate, 2'-ethoxy-2-ethoxyethyl(meth)acrylate, ethyleneglycol di(meth)acrylate, diethyleneglycol di(meth)acrylate, triethyleneglycol di(meth)acrylate (TEGDMA), tetraethyleneglycol di(meth)acrylate, polyethyleneglycol mono-(meth)acrylate, polyethyleneglycol di(meth)acrylate, polypropyleneglycol mono-(meth)acrylate, polypropyleneglycol di(meth)acrylate, polytetramethyleneglycol mono-(meth)acrylate, polytetramethyleneglycol di(meth)acrylate, hexanediol di(meth)acrylate, trimethyloylpropane tri(meth)acrylate, ethoxylated trimethyloylpropane tri(meth)acrylate, UDMA (reaction product of 2-hydroxyethyl methacrylate with 2,4,4-trimethylhexane diisocyanate), 2,2-bis[4-(2-hydroxy-3-methacryloylpropoxy)-phenyl]-propane (Bis-GMA), ethoxylated bisphenol A dimethacrylate ("EBPADMA-n", n=total number of moles of ethylene oxide in the molecule, with 2-20 units being preferred), tetrahydrofurfuryl (meth)acrylate, N,N'-methylenebis(acrylamide), N,N'-ethylenebis(acrylamide), N,N'-butylenebis(acrylamide), or a mixture thereof. In one embodiment, the bonding sealant composition comprises at least one polymerizable monomer having at least one hydroxyl group. Examples of hydroxyl-containing polymerizable monomers include, but are not limited to, hydroxyethyl (meth)acrylate, hydroxypropyl (meth)acrylate, hydroxybutyl (meth)acrylate, glycerol di(meth)acrylate, glycerol mono(meth)acrylate, and 2,2-bis[4-(2-hydroxy-3-methacryloylpropoxy)-phenyl]-propane (Bis-GMA). Examples of ethylenically unsaturated monomers comprising two or more methacrylate groups (bis-methacrylate monomer) include bisphenol A ethoxylate dimethacrylate (Bis-EMA), 1,6-bis-(methacryloyloxy-2-ethoxycarbonylamino)-2,4,4-trimethylhexane, which is called the urethane-dimethacrylate monomer (UDMA), 2,2-bis-[4-(2-hydroxy-3-methacryloxypropoxy)phenyl]propane (Bis-GMA, bisphenol A glycerolate dimethacrylate), and triethylene glycol dimethacrylate (TEGDMA).

Optionally, the bonding sealant composition forming the layer 30 may further comprise a filler. Examples of fillers include, but are not limited to, one filler composition or a combination of the following filler compositions: inorganic metal, salt, oxide, fluoride, nitride, silicate glass, aluminosilicate glass, aluminoborosilicate glass, fluoroaluminosilicate glass, barium-containing glass, quartz, colloidal silica, precipitated silica, zirconia-silica, mixed oxides fused together, polymeric filler, and polymerized composite fillers with inorganic particles. In one embodiment, inorganic fillers for increased x-ray contrast ability include metals, salts, oxides, fluorides, silicate glass, aluminosilicate glass, aluminoborosilicate glass, and fluoroaluminosilicate glass containing one or more elements of high atomic number, such as Sr, Y, Zr, Ba, La, Hf, Zn, Bi, W, rare earth metals, and combinations of these. Specific compositional examples include barium sulfate, silver, strontium fluoride, barium fluoride, ytterbium fluoride, yttrium fluoride, barium tungstate, zinc oxide, bismuth(III) oxide, bariumaluminosilicate, bariumaluminoborosilicate, strontiumaluminosilicate, bariumfluoroaluminosilicate, strontiumfluoroaluminosilicate, strontiumzincfluoroaluminosilicate, zincaluminosilicate, etc. Fumed silica, colloidal silica, or precipitated silica can also be incorporated to improve the dispersion of the filler, as well as the rheological and handling properties of the composition. Examples of colloidal silicas are the Aerosil series such as OX-50, OX-130, and OX-200 silica sold by Degussa (Ridgefield Park, N.J.), and Cab-O-Sil M5 and Cab-O-Sil TS-530 silica sold by Cabot Corp (Tuscola, Ill.). The filler may also include nanoparticles, such as those obtained through a sol-gel process. Mixtures of different fillers can be used. For inorganic fillers, the surface of the filler may be treated or coated with a coupling agent, such as gamma-methacryloyloxypropyltrimethoxysilane (MPTMS), that enhances the interfacial bonding between the filler and resin matrix and improves mechanical properties.

In one embodiment, the mean particle size of the filler is less than 10 microns. In another embodiment, the mean particle size of the filler is less than 5 microns. In another embodiment, the mean particle size of the filler is less than 1 micron. For example, the filler may have an average particle diameter in a range of about 0.5 to about 0.9 µm, about 1.7 to about 2.3 µm; or about 8 to about 10 µm. Filler(s) having different sizes may be present in the bonding sealant composition. When present, an amount of filler(s) in the bonding sealant composition may be within a range of about 1% (w/w) to about 30.0% (w/w).

In one embodiment, the bonding sealant composition further includes a volatile solvent. Nonlimiting examples of useful solvents include methanol, ethanol, isopropanol, acetone, ethyl acetate, methyl ethyl ketone, or combinations thereof. Water may be present in the bonding sealant composition as a co-solvent.

The bonding sealant composition may further include one or more stabilizers. The stabilizer(s) may be present in the bonding sealant composition in an amount of from about 0 to about 5% (w/w), such as from about 0.001% (w/w) to about 2% (w/w), or from 0.01% (w/w) to about 1% (w/w).

In one embodiment, the bonding sealant composition is a commercially available orthodontic sealant, such as Ortho Solo^{™}, available from Kerr Corporation of Orange, Calif.

As shown in FIG. 3, the layer **32** may then be directly applied on the layer **30** in a separate application. The photo-curable orthodontic adhesive composition may be applied to layer **30** to form layer **32,** which chemically bonds to the layer **30** and also mechanically bonds to the orthodontic bracket **12.** The photo-curable orthodontic adhesive composition may include a resin, such as one or more (meth)acrylate-based resins described above. In an embodiment, the photo-curable orthodontic composition comprises at least one ethylenically unsaturated monomer comprising two or more methacrylate groups. Upon initiating radical polymerization, the methacrylate groups can chemically bond with the (meth)acrylate-based resin in the bonding sealant composition of layer **30.** Accordingly, the layer **32** may include a photo-initiator system (described below) to facilitate curing of the layer **32.** The compositions of layers **26** and/or **30** may also include a photo-initiator system.

The photo-curable orthodontic adhesive composition may further include a catechol methacrylate (CMA) monomer having the general Formula (I), a photo-initiator system, and a filler, each described above. The CMA monomer may be the same or different monomer to what is within the bonding primer composition and/or the bonding sealant composition, if present therein.

In accordance with embodiments of the invention, the photo-curable orthodontic adhesive composition is photopolymerizable, i.e., the hardenable component is photopolymerizable and the hardener includes a photo-initiator system, in which irradiation with actinic radiation initiates the polymerization (or hardening) of the adhesive. Such photopolymerizable compositions can be free radically polymerizable or cationically polymerizable.

Suitable photo-initiators (i.e., photo-initiator systems that include one or more compounds) for polymerizing free radically photopolymerizable compositions include binary and tertiary systems. Typical binary photo-initiators include a ketone sensitizer and an amine donor. Typical tertiary photo-initiators include a ketone sensitizer, an amine donor, and an iodonium salt, as described in U.S. Pat. No. 5,545,676 (Palazzotto et al.). Preferred photosensitizers are ketone donors (monoketones and diketones) that absorb some light within a range of 400 nm to 520 nm (preferably, 450 nm to 500 nm). More preferred compounds are alpha diketones that have some light absorption within a range of 400 nm to 520 nm (even more preferably, 450 to 500 nm). Preferred compounds are camphorquinone, benzil, furil, 3,3,6,6-tetramethylcyclohexanedione, phenanthraquinone, 1-phenyl-1,2-propanedione and other 1-aryl-2-alkyl-1,2-ethanediones, and cyclic alpha diketones. Most preferred is camphorquinone. Preferred amine donors include, but are not limited to, ethyl 4-(N,N-dimethylamino)benzoate, 4-(N,N-dimethylamino)benzoic acid, 4-(N,N-dimethylamino)benzonitrile, 4-(N,N-dimethylamino)benzaldehyde, 2-(ethylhexyl)-4-(N,N-dimethylamino)benzoate, N,N-dimethylaminoethyl methacrylate, N,N-dimethylaminophenethyl alcohol. Preferred iodonium salts are the diaryl iodonium salts, e.g., diphenyliodonium chloride, diphenyliodonium hexafluorophosphate, diphenyliodonium tetrafluoroborate, and tolylcumyliodonium tetrakis(pentafluorophenyl)borate. Other suitable tertiary photo-initiator systems useful for photopolymerizing cationically polymerizable resins are described, for example, in U.S. Pat. No. 6,765,036 (Dede et al.). A typical formulation for the photo-curable orthodontic adhesive composition is shown in Table 2, below.

**TABLE 2: Typical formulation for the photo-curable orthodontic adhesive composition:**

| **Chemical** | **Example** | **Weight %** |
|---|---|---|
| CMA | C10-CMA | 0.01 to 1 |
| Bis-methacrylate monomer 1 | Bis-GMA | 1.58 to 25 |
| Bis-methacrylate monomer 2 | Bis-EMA | 5 to 25 |
| Iodonium salt | DPI | 0.05 to 0.5 |
| Ketone sensitizer | Camphorquinone or CQ | 0.05 to 0.5 |
| Amine donor | EHDMAB | 0.05 to 1 |
| Filler 1 | 0.7 µm barium glass filler | 1 to 20 |
| Filler 2 | 2.0 µm barium glass filler | 0.5 to 20 |
| Filler 3 | 9.0 µm barium glass filler | 35 to 70 |

In an embodiment, the photo-curable orthodontic adhesive comprises 0.02% (w/w) of CMA; 28.08 of a mixture of two bis-methacrylate monomers; a photo-initiator system comprising 0.14% (w/w) of iodonium salt, 0.05% of ketone sensitizer, and 0.1% (w/w) amine donor; and 71.61% (w/w) fillers.

In the case of the layer **32,** which includes the photo-initiator system, the orthodontic bracket **12** may be pressed against the composite layer **26, 30,** and **32** shown in FIG. 3. The adhesive layer **32** may then be cured by exposing it to light, such as visible blue light (e.g., wavelengths of about 450 nm to about 475 nm). This photo-curing process cures at least the layer **32.** By way of further example, each of the layers **26, 30,** and **32** (FIG. 3) may be cured at the same time or at different times. The timing of each cure depends on the preferences of the clinician. For example, a clinician may prefer to partially cure the layer **30** to make it tackier and then apply the remaining layer **32** with a final cure of each of all the layers together. When the composite layers are cured, the orthodontic adhesive system **10** bonds the orthodontic bracket **12** to the tooth surface **22.**

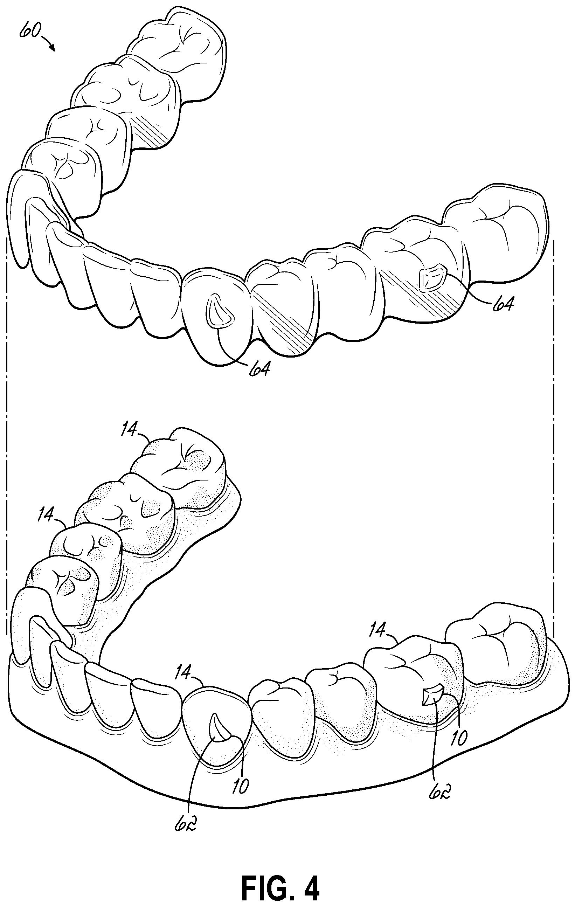

With reference now to FIG. 4, in another embodiment, the orthodontic adhesive system **10** may be utilized to facilitate orthodontic treatment with other orthodontic appliances, such as an aligner **60.** The aligner **60** may be configured to fit over and interact with one or more aligner attachments **62** bonded to one or more teeth **14.** Each aligner attachment **62** may be a structure of predetermined shape of the orthodontic adhesive system **10.** That is, the orthodontic adhesive system **10** may be formed into a rectangular, square, circular, ellipsoidal or triangular-shaped attachment that is bonded to the tooth surface in a manner similar to that described above respect to the orthodontic brackets **12** (FIG. 1). The aligner attachment **62** may be formed completely of the adhesive layer **32.**

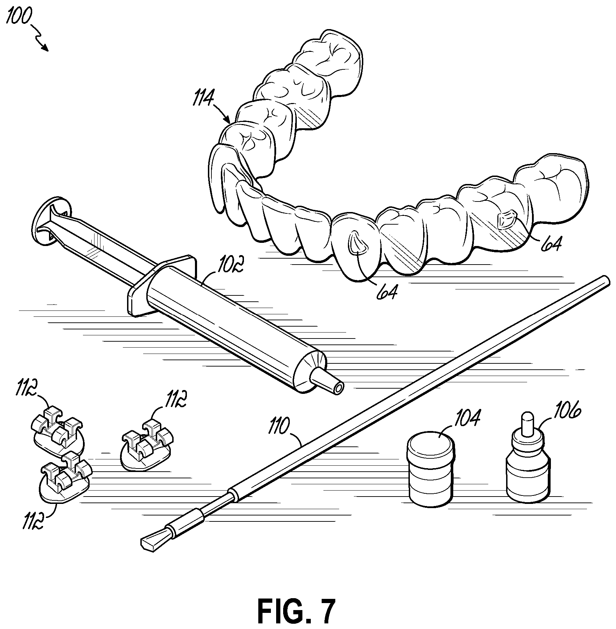

The aligner **60** may be configured with a corresponding receptacle **64** that engages the aligner attachment **62** during orthodontic treatment. Advantageously, each of the aligner attachments **62** may be easily bonded to the tooth surface prior to treatment with an aligner **60.** The aligner attachments **62** may be applied to the tooth with the use of a template **114** (see Fig. 7) that allows the clinician to more easily locate the aligner attachment **62** on the patient's tooth. An accurately placed aligner attachment **62** may interact with the corresponding receptacle **64** in the aligner **60.**

Advantageously, the orthodontic adhesive system **10,** which includes the catechol methacrylate monomer having the general Formula (I) in both the bonding primer composition and the photo-curable orthodontic adhesive composition provides superior bonding strength of the orthodontic bracket **12** or the aligner attachment **62** to the tooth surface **22.** Due to the catechol methacrylate monomer in the formulation, the orthodontic adhesive system **10** is moisture tolerant and can bond in moist and wet environments. Traditionally, doctors ensure that the teeth are isolated from any kind of water, saliva, or moisture, but the orthodontic adhesive system **10** obviates the need to dry the tooth in order to bond.

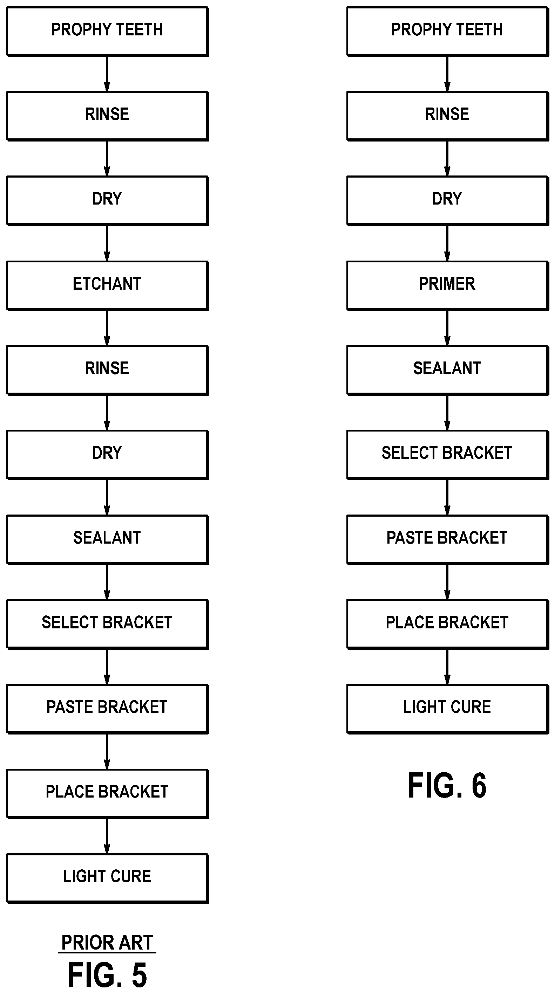

In reference to FIG. 5, the prior art methods of adhering an orthodontic device to a tooth typically require prophy teeth, rinsing, drying, etching, rinsing, drying, applying a sealant, selecting a bracket, paste bracket, place bracket, and then light curing. Advantageously, the orthodontic adhesive system **10** described herein obviates any need for use of an etchant and thus eliminates the added steps of etching and rinsing of the etchant.

In reference to FIG. 6 and in accordance with an embodiment of the invention, a method of adhering an orthodontic device to a tooth is provided. The method comprises providing a system comprising a bonding primer composition, a bonding sealant composition, and the photo-curable orthodontic adhesive composition. In one embodiment, a clinician then utilizes the system in an order specific to build a system of layers described above with reference to FIG. 3, for example, on a patient's tooth. As an example, the bonding primer composition is applied to a cleaned surface of the tooth **14** to form the layer **26.** The bonding primer composition comprises a catechol methacrylate monomer and a volatile solvent. After application of the layer **26,** the bonding sealant composition is applied to the primer layer **26** to form a sealant layer **30** thereon. The bonding sealant composition comprises at least **25** weight percent of 2-hydroxyethyl methacrylate, and a filler. After application of layer **30** the photo-curable orthodontic adhesive composition is applied to the sealant layer **30** and/or to the orthodontic device. The orthodontic device is then affixed to the tooth with the primer layer **26,** sealant layer **30,** and the photo-curable orthodontic adhesive composition (shown as the layer **32** in FIG. 3) situated between the tooth and the orthodontic device. The method may further include performing prophylaxis on the surface of the tooth with an abrasive pumice prior to applying the first layer **26;** and removing the abrasive pumice from the surface of the tooth by rinsing with water and drying the tooth prior to applying the bonding primer composition.

In an embodiment of the invention, the photo-curable orthodontic adhesive may be used in a system along with the bonding primer composition and the bonding sealant composition. In an embodiment, the system may further include a debonding composition. The system may be void of any etchant composition. The system may further include an orthodontic device, such as orthodontic bracket **12,** on which the orthodontic adhesive is pre-applied. The system may include a bubble pack in which the brackets **12** are individually disposed. The clinician may remove the orthodontic brackets **12** individually from the packaging in a particular order and press them to the patient's teeth. The clinician may then cure the pre-applied adhesive with light, such as blue light.

The orthodontic adhesive system **10** may be provided as a kit **100.** In reference to FIG. 7, each of the photo-curable orthodontic adhesive, the bonding primer composition, and the bonding sealant composition may be packaged separately into individual containments. In an embodiment, the photocurable orthodontic adhesive may be packaged in a syringe **102;** the bonding primer composition may be packaged in a resealable vial **104;** and the bonding sealant composition may be packaged in a dispensing bottle **106.** The kit **100** may further comprise a brush applicator **110,** as well as orthodontic brackets **112** and/or the template **114.**

### EXAMPLE

In order to facilitate a more complete understanding of the embodiments of the invention, the following non-limiting example is provided.

The enamel of a tooth is pumiced using an oil-free pumice paste. After the pumice is rinsed and dried. The Bonding Primer Composition (Table 1) is applied to the enamel with a microbrush. The Bonding Sealant Composition (Ortho Solo^{™}) is applied afterwards. The Photo-curable Orthodontic Adhesive Composition (Table 2) is applied either to a bracket pad or an aligner template to create aligner attachments. The bracket or template is applied to the tooth and light cured.

### Wire Shear Test for bracket:

Equipment: Instron model 5944 or 5544 with Bluehill Universal software 500N static load cell; Debonding fixture: FIX-5131; Scalar; Forceps; Preppies pumice paste; Potted bovine teeth; Air hose with pressure gauge; Washer, nut, and screw.

Procedure: Pumice a potted bovine tooth using a prophy cup. Rinse off any remaining pumice and air dry. Apply one coat of Bonding Primer Composition (Table 1) to the tooth. Apply one coat of Bonding Sealant Composition (Ortho Solo^{™}) to the tooth. Dispense Photo-curable Orthodontic Adhesive Composition (Table 2) onto the pad of a bracket. Use a spatula to press the adhesive into the pad. Place the bracket onto the tooth and press the bracket firmly onto the enamel. Remove any flash around the perimeter of the pad using a scalar. Light cure for 10 seconds for ceramic brackets and 20 seconds for metal brackets. Store in a container filled with DI water and place in an oven at 37°C for 24 hours. Make sure that 500 N load cell is installed on the crosshead. Install pneumatic grip to the load cell as shown below. Affix the debonding fixture onto the plate. Secure a 0.020" round archwire using a washer, screw, and nut so that it creates a loop. Place the loop into the pneumatic grip. Insert the test samples into the holder, which holds 5 samples. Insert the sample holder in the debonding fixture. Orient the sample so that the bottom of the wire loop is under the tie wing around the base of the bracket. Ensure archwire slot is horizontally oriented. Ensure the arch wire loop is positioned at the base of the bracket so it pulls upward at a 90° angle. Press "Zero Extension" and "Balance Load". Place the safety shield on and press START button. Wait till test is completed. Acceptance criteria for Wire shear - brackets: Average (36 samples) results ≥ 7.0 MPa; Individual results ≥ 4.0 MPa.

### Knife Shear Test for aligner attachment:

Equipment: Instron model 5944 or 5544 with Bluehill Universal software 500 N static load cell; Debonding fixture with sample holders; Knife fixture "FIX-5040"; Air hose with pressure gauge; Aligner attachment mold, size 4mm x 4mm x 2mm; Potted bovine teeth; and Preppies pumice paste.

Procedure: pumice a potted bovine tooth using a prophy cup. Rinse off any remaining pumice and air dry. Apply one coat of the Bonding Primer Composition (Table 1) to the tooth. Apply one coat of the Bonding Sealant Composition (Ortho Solo^{™}) to the tooth. Fill an aligner attachment mold with the Photo-curable Orthodontic Adhesive Composition (Table 2). Place the mold over the tooth and light cure for 10 seconds. Remove the mold. Store the potted tooth in a container filled with DI water and place in an oven at 37°C for 24 hours. Open Bluehill Universal software and select "Test" then "Knife Test" method. Attach the 500N load cell to the Instron and calibrate. Be sure that nothing is touching the Instron at the moment of calibration. Attach the knife fixture to the load cell. Place samples in the sample holder. Attach the fixture to the Instron base so that the knife fixture is perpendicular to the attachment. Press "Balance All" on the Bluehill software then press "Start." The Instron should record the maximum load upon debond. Repeat for the rest of the samples. Acceptance criteria for Knife shear - aligner attachments: Average (36) results ≥ 6.0 MPa; Individual results ≥ 3.5 MPa.

Unless otherwise explained, all technical and scientific terms used herein have the same meaning as commonly understood by one of ordinary skill in the art to which this disclosure belongs. In case of conflict, the present specification, including explanations of terms, will control. The singular terms "a," "an," "at least one," and "the" include plural referents unless context clearly indicates otherwise. Similarly, the word "or" is intended to include "and" unless the context clearly indicates otherwise. The term "comprising" means "including;" hence, "comprising A or B" means including A or B, as well as A and B together. While the invention has been illustrated by the description of one or more embodiments thereof, and while the embodiments have been described in considerable detail, they are not intended to restrict or in any way limit the scope of the appended claims to such detail. Additional advantages and modifications will readily appear to those skilled in the art. The invention in its broader aspects is therefore not limited to the specific details, representative product and/or method and examples shown and described. The various features of exemplary embodiments described herein may be used in any combination. Accordingly, departures may be made from such details without departing from the scope of the general inventive concept.

## Claims

1. A photo-curable orthodontic adhesive composition comprising:
a catechol methacrylate monomer;
at least one ethylenically unsaturated monomer comprising two or more methacrylate groups;
a photo-initiator system; and
a first filler.

2. The photo-curable orthodontic adhesive composition according to claim 1, wherein the catechol methacrylate monomer comprises 10-((3-(3,4-dihydroxyphenyl)propanoyl)oxy)decyl methacrylate.

3. The photo-curable orthodontic adhesive composition according to preceding claim 1 to 2, wherein the at least one ethylenically unsaturated monomer comprising two or more methacrylate groups is selected from the group consisting of 2,2-bis-(4-(2-hydroxy-3-methacryloyloxypropoxy) phenyl) propane (Bis-GMA), triethyleneglycol dimethacrylate (TEGDMA), ethylene glycol dimethacrylate (EGDMA), ethoxylate bisphenol A dimethacrylate (Bis-EMA), urethane dimethacrylate (UDMA), anhydrous 4-methacryloxyethyl trimellitic anhydride (4-META), dipentaerythritol pentaacrylate monophospate, biphenyl dimethacrylate (BPDM), glycerolphosphate dimethacrylate (GPDM), propylene glycol dimethacrylate (PGDMA), and combinations thereof preferably the at least one ethylenically unsaturated monomer comprising two or more methacrylate groups comprises a combination of 2,2-bis-(4-(2-hydroxy-3-methacryloyloxypropoxy) phenyl) propane (Bis-GMA) and ethoxylate bisphenol A dimethacrylate (Bis-EMA).

4. The photo-curable orthodontic adhesive composition according to any preceding claim 1 to 3, wherein the photo-initiator system comprising a ketone sensitizer, an amine donor, and optionally an iodonium salt.

5. The photo-curable orthodontic adhesive composition according to claim 4, wherein the ketone sensitizer is selected from the group consisting of camphorquinone, 1-phenyl propane-1,2-dione, benzil, diacetyl, benzyl dimethyl ketal, benzyl diethyl ketal, benzyl di(2-methoxyethyl)ketal, 4,4,'-dimethylbenzyl dimethyl ketal, anthraquinone, 1-chloroanthraquinone, 2-chloroanthraquinone, 1,2-benzanthraquinone, 1-hydroxyanthraquinone, 1-methylanthraquinone, 2-ethylanthraquinone, 1-bromoanthraquinone, thioxanthone, 2-isopropyl thioxanthone, 2-nitrothioxanthone, 2-methyl thioxanthone, 2,4-dimethyl thioxanthone, 2,4-diethyl thioxanthone, 2,4-diisopropyl thioxanthone, 2-chloro-7-trifluoromethyl thioxanthone, thioxanthone-10,10-dioxide, thioxanthone-10-oxide, diphenyliodonium hexafluorophosphate, *p*-octyloxy-phenylphenyl iodonium hexafluoroantimonate, benzoin methyl ether, benzoin ethyl ether, isopropyl ether, benzoin isobutyl ether, benzophenone, bis(4-dimethylaminophenyl)ketone, 4,4,'-bisdiethylaminobenzophenone, and combinations thereof; wherein amine donor comprises a secondary or tertiary amine selected from the group consisting of triethanolamine, diethanolamine, methyl diethanolamine, 2-dimethylaminoethyl (meth)acrylate, 3-dimethylaminopropyl (meth)acrylate, 4-dimethylaminobutyl (meth)acrylate, 6-dimethylaminohexyl (meth)acrylate, 10-dimethylaminodecyl (meth)acrylate, 4-dimethylaminophenetyl alcohol, 2-(4-(dimethylamino)phenyl)ethanol, 2-(4-(dipropylamino)phenyl)ethanol, 2-ethylhexyl-4-(dimethylamino) benzoate, N,N-di(2-hydroxyethyl)-p-toluidine, N,N-di(2-hydroxypropyl)-p-toluidine, N,N-dimethyl-p-toluidine, N,N-dipropyl-p-toluidine, N,N-diethyl-p-toluidine, N,N-diethoxyethyl-p-toluidine, N,N-dibutoxy ethyl-p-toluidine, N,N-di(polyoxyethylene)oxyethyl-p-toluidine, hexamethylenediamine, a dimethylamine aqueous solution, pentamethylenediamine, diethylamine, ethylenediamine, 2-aminoethanol, triethylamine, 2-dimethylaminoethanol, and combinations thereof and wherein the iodonium salt is present and selected from the group consisting of tolylcumyliodonium tetrakis(pentafluorophenyl)borate, tolylcumyliodonium tetrakis(3,5-bis(trifluoromethyl)-phenyl)borate, diphenyliodonium chloride, diphenyliodonium hexafluorophosphate, diphenyliodonium hexafluoroantimonate, diphenyliodonium tetrafluoroboarate, and combinations thereof.

6. The photo-curable orthodontic adhesive composition according to any preceding claim 1 to 5, wherein the first filler is selected from the group consisting of inorganic metal, salt, oxide, fluoride, or nitride; silicate glass; aluminosilicate glass; aluminoborosilicate glass; fluoroaluminosilicate glass; barium-containing glass; quartz; colloidal silica; precipitated silica; zirconia-silica; mixed oxides fused together; polymeric filler; polymerized composite fillers with inorganic particles; and combinations thereof.

7. The photo-curable orthodontic adhesive composition according to claim 6, wherein first filler comprises a barium-containing glass filler comprising:
a first barium glass filler having an average particle diameter in a range of about 0.5 to about 0.9 µm;
a second barium glass filler having an average particle diameter in a range of about 1.7 to about 2.3 µm; and
a third barium glass filler having an average particle diameter in a range from about 8 to about 10 µm.

8. The photo-curable orthodontic adhesive composition according to any preceding claim 1 to 7, wherein the catechol methacrylate monomer comprises 10-((3-(3,4-dihydroxyphenyl)propanoyl)oxy)decyl methacrylate;
wherein the at least one ethylenically unsaturated monomer comprising two or more methacrylate groups comprising a combination of 2,2-bis-(4-(2-hydroxy-3-methacryloyloxypropoxy) phenyl) propane (Bis-GMA) and ethoxylate bisphenol A dimethacrylate (Bis-EMA);
wherein the photo-initiator system comprising a ketone sensitizer, an amine donor, and an iodonium salt; and
wherein the first filler comprising a barium-containing glass filler comprising:
a first barium glass filler having an average particle diameter in a range of about 0.5 to about 0.9 µm;
a second barium glass filler having an average particle diameter in a range of about 1.7 to about 2.3 µm; and
a third barium glass filler having an average particle diameter in a range from about 8 to about 10 µm.

9. The photo-curable orthodontic adhesive composition according to any preceding claim 1 to 8, wherein the photo-initiator system comprising camphorquinone, 2-ethylhexyl-4-(dimethylamino) benzoate, and diphenyliodonium hexafluorophosphate.

10. The photo-curable orthodontic adhesive composition according to any preceding claim 1 to 9, comprising:
0.01 to 1.0 weight percent of the catechol methacrylate monomer;
5.0 to 25.0 weight percent of the at least one second ethylenically unsaturated monomer comprising two or more methacrylate groups;
0.1 to 5.0 weight percent of the second photo-initiator system; and
50.0 to 80.0 weight percent of the first filler,
wherein each weight percent is based on the entire weight of the photo-curable orthodontic adhesive composition.

11. An orthodontic adhesive system for adhering an orthodontic device to a tooth comprising:
a) a bonding primer composition comprising a second catechol methacrylate monomer and a volatile solvent;
b) a bonding sealant composition comprising at least 25 weight percent of 2-hydroxyethyl methacrylate and a second filler; and
c) the photo-curable orthodontic adhesive composition according to any one of claims 1-10,
wherein the orthodontic adhesive system is void of any etchant composition; and
wherein the first and second catechol methacrylate monomer are the same or different.

12. The orthodontic adhesive system for adhering an orthodontic device to a tooth according to claim 10, wherein the first and second catechol methacrylate monomer comprises 10-((3-(3,4-dihydroxyphenyl)propanoyl)oxy)decyl methacrylate.

13. An orthodontic adhesive system for adhering an orthodontic device to a tooth, comprising:
a syringe comprising the photo-curable orthodontic adhesive composition according to any one of claims 1-10;
a first container comprising a bonding primer composition comprising a second catechol methacrylate monomer and a volatile solvent;
a second container comprising a bonding sealant composition comprising at least 25 weight percent of 2-hydroxyethyl methacrylate, and a second filler; and
optionally, a brush applicator and/or an orthodontic device.

14. A method of adhering an orthodontic device to a tooth with an orthodontic adhesive system including a bonding primer composition, a bonding sealant composition, and the photo-curable orthodontic adhesive composition according to any one of claims 1-10, the method comprising:
applying the bonding primer composition to a cleaned surface of the tooth, wherein the bonding primer composition comprising a second catechol methacrylate monomer and a volatile solvent to form a primer layer on the cleaned surface of the tooth;
applying the bonding sealant composition to the primer layer to form a sealant layer thereon, wherein the bonding sealant composition comprising at least 25 weight percent of 2-hydroxyethyl methacrylate, and a second filler;
applying the photo-curable orthodontic adhesive composition to the sealant layer previously applied to the primer layer, and/or applying the photo-curable orthodontic adhesive composition to the orthodontic device; and
affixing the orthodontic device to the tooth with the primer layer, sealant layer, and the photo-curable orthodontic adhesive composition situated between the tooth and the orthodontic device;
wherein the first and second catechol methacrylate monomer are the same or different.

15. The method of adhering the orthodontic device to the tooth according to claim 14, further comprising:
performing prophylaxis on the surface of the tooth with an abrasive pumice prior to applying the first layer; and
removing the abrasive pumice from the surface of the tooth by rinsing with water and drying the tooth.
